# EUROPEAN PATENT APPLICATION

(11) **EP 2 305 310 A1**
(43) Date of publication of application: **06.04.2011**
(21) Application number: 09382185.8
(22) Date of filing: 25.09.2009
(51) Int. Cl.: A61K 47/48, A61K 49/18

(54) **Gold -coated magnetic glyconanoparticles functionalised with proteins for use as diagnostic and therapeutic agents**

(71) Applicant: Asociación Centro de Investigación Cooperativa en Biomateriales - CIC biomaGUNE, 2008 San Sebastián (ES)
(72) Inventor: Penadés, Soledad, 20008, San Sebastián (ES); García, Isabel, 20009, San Sebastián (ES); Gallo, Juan, 20009, San Sebastián (ES)
(74) Representative: ZBM Patents

(57) **Abstract**

Gold-coated nanoparticles comprising:
a) a magnetic core of XFe₂O₄ wherein X is a metal selected from the group consisting of Fe, Mn, and Co;
b) carbohydrates covalently coupled through a spacer to the gold-coated nanoparticle, wherein said spacer has a tiol linking it to the nanoparticle; and
c) an immunoglobulin-binding protein coupled to the gold-coated nanoparticle through an amphiphilic molecule, comprising a functional group capable of coupling the protein and through a thiol group which is attached to the gold coating of the nanoparticle, where a inmunoglobulin-binding protein can be coupled to further bioconjugation with antibodies. Said nanoparticles have been found to be useful as as contrast agents by MRI.

## Description

The present invention relates to superparamagnetic gold-coated nanoparticles containing a magnetic core of iron oxide ferrites, namely XFe₂O₄ (X=Fe, Mn or Co) and functionalised with immunoglobulin-binding proteins for attaching antibodies thereto; their preparation process and their uses.

### BACKGROUND ART

Among a variety of nanoparticles, magnetic nanoparticles, which can be manipulated using magnetic fields, have shown a great potential in biomedical applications, i.e. as magnetic resonance image (MRI) contrast agents.

Superparamagnetic iron oxide ferrites XFe₂O₄ (X=Fe, Mn or Co) nanoparticles are known and also their use as MRI contrast agents for in vivo applications, i.e. diagnosis. Target-specific monoclonal antibodies and peptides coupled to said nanoparticles are individually prepared to visualise pathologic areas such as tumours.

The gold coated ferrite core nanoparticles disclosed in the state of the art are prepared using non-polar solvents. Said nanoparticles are then extracted from the non-polar solvent. To provide solubility in water and prevent aggregation of nanoparticles a variety of amphiphilic molecules are used, for instance polyethylene glycol (PEG); these amphiphilic molecules are also used as linkers (e.g. WO 2008/048074). The nanoparticles have then been used as diagnostic agents by coupling a target-specific ligand on their surface. The target specific ligand is typically conjugated to the nanoparticles using dimercaptosuccinic acid (DMSA); the DMSA binds the ligand, e.g. antibody through its carboxylic group. Some of the nanoparticles disclosed in the art have a gold cover, said cover offers the possibility to anchor organic molecules to the nanoparticles through a S-Au link; however, said nanoparticles cannot be used as universal platform to attach the target-specific molecules of interest in an easy and biocompatible way.

Lim et al., in "Simultaneous intracellular delivery of targeting antibodies and functional nanoparticles with engineered proteing G system", Biomaterials (2009) 30:1197-1204, disclose gold-coated iron oxide nanoparticles functionalised with an engineered protein G, which contains an hexahistidine tag. However, said nanoparticles have been solubilised using PEG, which may induce an undesirable immuno response. And moreover, in order to covalently linked the protein G to the nanoparticle, an engineered protein G comprising an hystidine tag region has to be produced. The genetically modified protein G is then binded to the nanoparticle through metal ions such as Ni(II) and Cu(II) that are covering the nanoparticle.

There is, hence, the need to develop gold-coated superparamagnetic nanoparticles to be used as diagnostic agents, in particular, a nanoparticle structure that permits a universal platform to attach immunoglobulin-binding proteins on the coating, to be used in diagnostic, e.g. as MRI contrast agents, which nanoparticles have a high degree of biocompatibility and biostability.

### SUMMARY OF THE INVENTION

The inventors have found biofunctional glyconanoparticles, with high biostability and biocompatibility, being highly soluble in water and in a physiological buffer, which are a general platform to capture well-oriented antibodies through covalent immobilisation of immunoglobulin-binding proteins.

Thus, a first aspect of the present invention relates to a gold-coated nanoparticle comprising: a) a magnetic core of XFe₂O₄ wherein X is a metal selected from Fe, Mn, and Co; b) carbohydrates covalently coupled through a spacer to the gold-coated nanoparticle, wherein said spacer has a tiol linking it to the nanoparticle; and, c) an immunoglobulin-binding protein coupled to the gold-coated nanoparticle through an amphiphilic molecule comprising a functional group capable of coupling the protein and a thiol group which is attached to the gold coating of the nanoparticle.

Another aspect of the present invention relates to a method for the preparation of said nanoparticles. The method comprising: (a) coating a magnetic core of XFe₂O₄ wherein X is a metal selected from the group consisting of Fe, Mn, and Co by contacting the core with Au(Acetyl)₃ (Au(Ac)) in the presence of at least one surfactant; (b) mixing a non-polar solution of the gold-coated nanoparticles obtained in step (a) with an aqueous solution of glycoconjugates and amphiphilic molecules, the amphiphilic molecules comprising a functional group capable of coupling the protein and a thiol group which is attached to the gold coating of the nanoparticle; and (c) covalently coupling an immunoglobulin-binding protein to the nanoparticles.

Another aspect of the present invention relates to the use of the nanoparticles as defined above as contrast agents in MRI. It also forms part of the invention a method for the diagnosis and treatment of cancer using the magnetic gold nanoparticles as defined above.

Finally, another aspect of the invention relates to a kit comprising any of the nanoparticles of the underlying invention.

### DETAILED DESCRIPTION OF THE INVENTION

According to the invention, the term XFe₂O₄@Au refers to nanoparticles having a core of XFe₂O₄ (X=Fe, Mn or Co) and a gold layer.

According to the invention, "glycoconjugate" is a carbohydrate covalently linked to a molecule. A spacer is, hence, a molecule covalently linked to the carbohydrate and which is used to couple it to the nanoparticle.

According to the invention, a glyconanoparticle refers to glycoconjugates covalently linked to a nanoparticle.

According to the invention, a linker is an amphiphilic molecule comprising a functional group capable of coupling the protein and a thiol group to be attached to the gold coating of the nanoparticle.

According to the invention, the term XFe₂O₄@Au @PG refers to XFe₂O₄@Au (X=Fe, Mn or Co) nanoparticles biofunctionalised with protein G.

According to the invention, the term XFe₂O₄@Au @PG@Ab refers to XFe₂O₄@Au (X=Fe, Mn or Co) nanoparticles biofunctionalised with protein G and conjugated to antibodies.

As mentioned above, the inventors have disclosed glycoconjugated nanoparticles functionalised with immunoglobulin-binding proteins. The monodispersed gold-coated nanoparticle of the invention comprises: a) a magnetic core of XFe₂O₄ wherein X is a metal selected from Fe, Mn, and Co; b) carbohydrates covalently coupled through a spacer to the gold-coated nanoparticle, wherein said spacer has a tiol linking it to the nanoparticle; and, c) an immunoglobulin-binding protein coupled to the gold-coated nanoparticle through an amphiphilic molecule comprising a functional group capable of coupling the protein and a thiol group which is attached to the gold coating of the nanoparticle.

Also as mentioned above the inventors have disclosed a method of preparing the above gold-coated nanoparticles. The method comprises: (a) coating a magnetic core of XFe₂O₄ wherein X is a metal selected from the group consisting of Fe, Mn, and Co by contacting the core with Au(Ac)₃ in the presence of at least one surfactant; (b) mixing a non-polar solution of the gold-coated nanoparticles obtained in step (a) with an aqueous solution of glycoconjugates and amphiphilic molecules, the amphiphilic molecules comprising a functional group capable of coupling the protein and a thiol group which is attached to the gold coating of the nanoparticle; and, (c) covalently coupling an immunoglobulin-binding protein to the nanoparticles. It is also part of the present invention a method comprising the above steps and further comprising coupling a protein, a peptide, an antibody or a biomarker to the immunoglobulin-binding protein.

In a preferred embodiment, the magnetic core is Fe₃O₄. In a more prefered embodiment, the gold-coated Fe₃O₄ nanoparticles have a core size between 4 and 12 nm, and a gold-coated core size between 6 and 14 nm.

In another preferred embodiment, the magnetic core is MnFe₂O₄ or CoFe₂O₄. In another more preferred embodiment, the gold-coated MnFe₂O₄ and CoFe₂O₄ nanoparticles have a core size between 6 and 12 nm, and a gold-coated core size between 12 and 18 nm.

The process of gold coating carried out to obtain the gold-nanoparticles of the present invention allows to control the size of the gold shell. Example 5 illustrates how the process of coating is monitored.

For in vivo applications, the original hydrophobic coating (with at least one surfactant) of the monodispersed XFe₂O₄@Au nanoparticles, only soluble in non polar solvents, is changed into a hydrophilic and biocompatible coating by ligand exchange with a mixture of functional linkers and glycoconjugates.

In a preferred embodiment, the surfactants used are oleic acid and oleylamine. A reducing agent is also needed for the gold coating of the nanoparticles. In a preferred embodiment, 1,2-hexadecanediol is used as reducting agent. Regarding the glycoconjugates, any carbohydrate can be glycoconjugated to the nanoparticles using a spacer. The person skilled in the art knows how to conjugate the carbohydrates to the nanoparticles. In a preferred embodiment, the carbohydrate is any biological significant oligosaccharide. Preferred carbohydrates used in the present invention are lactose (Lacto), glucose (Glc) or glucosamine (GIcNAc) but any conjugate of biological significant glycan can also be used. Preferred glycoconjugates (including the spacer) used in the present invention are those corresponding to the following formula:

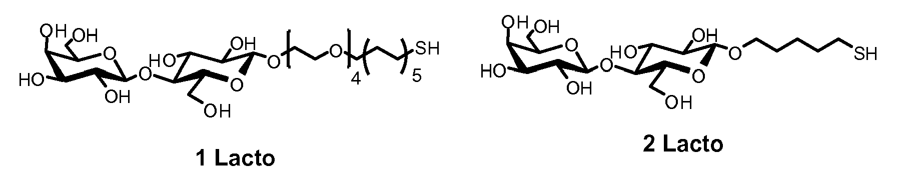

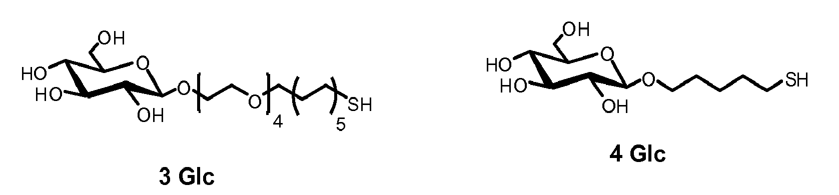

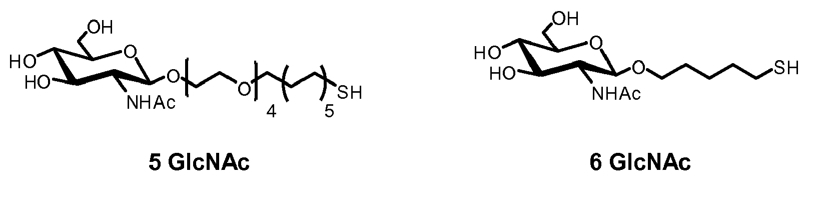

According to the invention, the terms glycoconjugates 1, 2, 3, 4, 5, and 6, refer to compounds having the above formula 1 Lacto, 2 Lacto, 3 Glc, 4 Glc, 5 GIcNAc and 6 GIcNAc, respectively.

Aqueous solutions of linkers ending in a thiol group and in activated functional groups, and/or glycoconjugates can be mixed, for instance, with hexane solutions of XFe₂O₄@Au to obtain the nanoparticles of the present invention. Linkers of different length and nature can be used.

The process of the invention can, hence, provide nanoparticles with different density on ligands, i.e. glycoconjugates and linkers. To prepare nanoparticles of different density, aqueous solutions containing a mixture of said glycoconjugates and linkers can be shacked together with the hexane soluble nanomaterial. The composition of the ligands on the resulting magnetic nanoparticles can be determined by integration of the 1 H-NMR spectrum before and after the ligand exchange.

In a preferred embodiment, a structure of glyconanoparticles having different density of carbohydrates and linkers is chosen. In another preferred embodiment, lacto-, gluco- and glucosamine-conjugates are selected to obtain XFe₂O₄@Au glyconanoparticles. In a preferred embodiment, the specific lacto-, gluco- and glucosamine-conjugates used, have the above disclosed formulas.

When the glycoconjugates used to prepare the nanoparticles are those corresponding to the above disclosed formulas 2, 4 and 6, the hexane is evaporated in a slowly way in order to force the molecules into water. This transfer process had 100% efficiency and no residue in the hexane phase is observed. However, to remove possible larger contaminants the solutions are passed through a 0.2-µm Nylon syringe filter.

In a preferred embodiment the linker has the following formula: X-(EG)₃-(CH₂)₁₁-SH, wherein EG is ethylene glycol and X is the functional group.

In a preferred embodiment, the linker used has a carboxylic acid, malemide or isothiocyanate as the ending functional group.

According to the invention, the term linker 7 refers to a compound having the following formula:

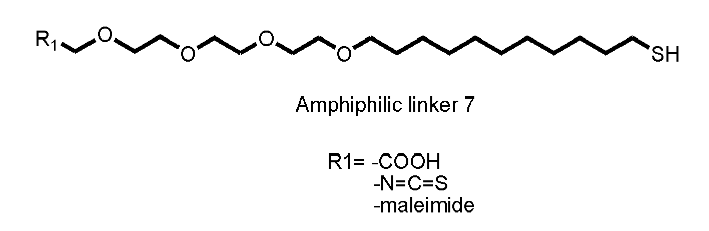

The process of synthesis of water soluble hybrid nanoparticles Fe₃O₄@Au (glycoconjugates 2, 3 or 4)₅₀ (linker 7)₅₀ is illustrated in Example 6.

The nanoparticles obtained after the above phase transfer, can be purified by lyophilisation and then co-dissolution in water. Generally, they are washed several times with magnetic separation and finally all the nanoparticles can be purified by dialysis.

The resulting glyconanoparticles are very soluble in water and physiological buffers. They do not precipitate in water over a wide pH range of 2-10, nor when dissolved in serum. The pH can be adjusted, for instance, with HCI or NaOH.

The ligand's shell provides multiple functional groups for further bioconjugation of important biomarkers like proteins, peptides and others. The XFe₂O₄@Au nanoparticles disclosed in the present invention can be biofunctionalised by covalently coupling a non-engineered immunoglobulin-binding protein using a linker ended in a functional group.

In a particular embodiment, the biofunctionalisation of the nanoparticles is carried out using a linker ended in a carboxylic acid; the protein is, succinimidylated, purified and co-dissolved in PBS.

In another embodiment of the present invention, the linker ends in an isothiocyanate. In a further embodiment of the present invention, the linker ends in a maleimide.

In a preferred embodiment, the immunoglobulin binding protein is protein G. This protein selectively and non-covalently immobilises constant fragments of immunoglobulin G (IgG) from diverse mammals constituting the perfect anchoring platform to capture oriented antibodies. The protein G immobilisation technique shows good reproducibility and coverage versus a disordered orientation of IgG molecules on the surface of the nanoparticle.

In another embodiment of the invention, the immunoglobulin binding protein is protein A.

In a particular embodiment, the protein G or protein A or the linker (preferrably the carboxylic acid group) have a fluorophore molecule. In a preferred embodiment the fluorophore molecule emits in the Red region (600nm<λ<750nm) or the Near-infraRed region (750nm<λ <900nm).

Once, the immunoglobulin binding protein is covalently linked, incubations with any antibody can be succesfully conjugated to those biofunctionalised nanoparticles for targeting in vivo specific organs or cells, to be used as biological markers or in hyperthermia treatments. Similarly as immunoglobulin binding proteins are covalently linked, peptides, other proteins, specific ligands or even molecular markers of different diseases can be also successfully conjugated. The hybrid nature of the nanoparticles of the present invention provides new oportunities as contrast agents by MRI and besides the gold layer leads other advantages: optical contrast enhancement, low toxicity and easy conjugation. The oriented immobilisation of monoclonal antibody on the magnetic glyconanoparticles supplies a tool for several applications, as proteomics tool.

The nanoparticles of the present invention, where the immunoglobulin-binding protein has a fluorophore molecule, can be used as detection agents of non-labelled antibodies. In an alternative embodiment, the fluorophore molecule is directly coupled to the -COOH group of the linker molecule.

For the characterisation of the formation of the full complexes comprising the biofunctionalised glyconanoparticles of the present invention, various techniques can be used. As a way of example, the UV-Vis spectra could be recorded from the Fe₃O₄@Au colloidal solution before and after conjugation with the antibody. The amount of antibody present in the washings, after the incubation, can also be measured using the Bradford method. Example 9 illustrates these techniques.

The biofunctionality of the molecules (i.e. antibodies) attached to the glyconanoparticles of the present invention can be tested using an immunoassay or quartz crystal microbalance with dissipation monitoring (QCM-D). In the immunoassay, the antibody system used can be goat-rabbit IgG as primary antibody to conjugate to the glyconanoparticles of the present invention and rabbit-antisheep labelled with HRP IgG as secondary antibody; controls experiments can be performed using the same glyconanoparticles without any antibody to evaluate non-specific interactions. Such immunoassay is illustrated in Example 10. Example 11 illustrates the QCM experiment.

After lyophilisation or centrifugation of biofunctionalised nanoparticles, the resulting nanoparticles usually show problems to redissolved in a physiological buffer, which is a previous step to use them as contrast agents (i.e. in MRI). However, the biofuntionalised nanoparticles of the underlying invention have high water solubility and are highly soluble in a phisiological buffer as well as biostability and biocompatibility.

The above biofunctionalised glyconanoparticles are useful contrast agents, since they are superparamagnetic at room temperature and present similar transverse relaxitivities to clinically used contrast agents like Endorem, Resovist or Sinerem; and as diagnostic and therapeutics agents, in general. In order to test the potential of the ferrites coated with gold of the present invention as contrast agents, longitudinal T1 and transversal T2 relaxation times can be measured and the calculated relaxivity values r2 can be compared with other contrast agents under clinical use, as illustrated in Example 13.

These nanopartices can be used in diagnostic methods. In a preferred embodiment, the diagnostic method is a method for cancer diagnosis comprising: administering cancer diagnostic agent into the human body, wherein said diagnostic agent comprises the nanoparticles of the present invention including the target-specific ligands as label cancer cells by binding selectively to said cancer cells, bind said cancer diagnostic agent with said cancer cell; and obtain magnetic resonance images using MRI system.

The nanoparticles can also be used in a method of treatment of cancer, the method comprising: administering a cancer therapeutic agent into the human body, wherein said cancer therapeutic agent comprises nanoparticles of the present invention and target, specific receptors bonded as to destroy cancer cells via heat generated by alternating electromagnetic fields (AEM); said cancer therapeutic agent to cancer cell; and deliver heat to the bound cancer cell.

In other words, the present invention provides a method for early stage diagnosis using target-specific ligands on the surface of said magnetic gold nanoshell to selectively accumulate the magnetic gold nanoshells on the cancer cell thereby achieving early stage diagnosis of the cancer cells using MRI, and selectively destroying the diagnosed cancer cells by AEM to said cancer cells.

According to the present invention, the magnetic gold nanoparticles are hence multifunctional nanomaterials used for MRI T2 contrast agents, MRI diagnosis of cancer cells and hyperthermia, and with simultaneous cancer diagnosis and therapy functionality great efficiency can be expected. When simultaneously using laser pulses, cancer cells can be destroyed in rapid time.

Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### BRIEF DESCRIPTION OF THE DRAWINGS

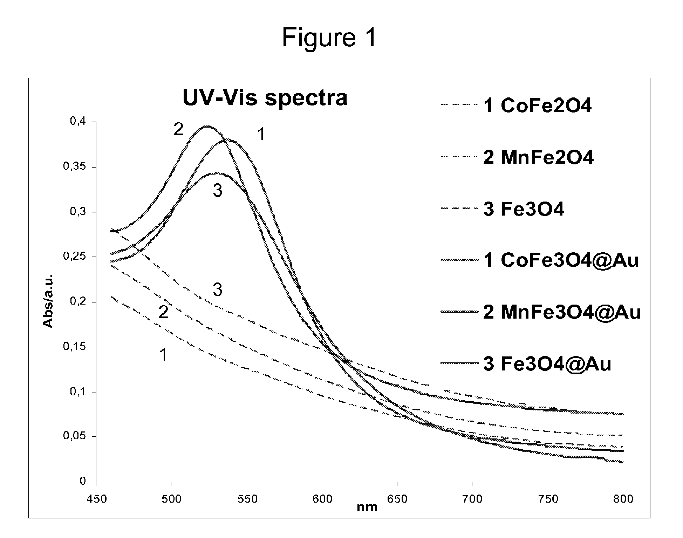

FIGURE 1. UV-Vis spectra of Fe₃O₄ (4nm), MnFe₂O₄ (6 nm) and CoFe₂O₄ (6 nm) before and after gold coating XFe2O4@Au.

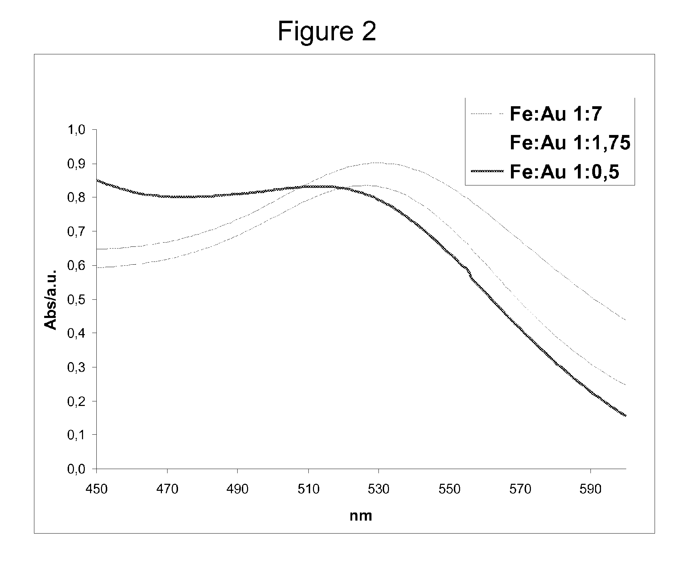

FIGURE 2. UV-Vis spectra of the core/shell Fe₃O₄@Au nanoparticles with three different Au ratios (A=Absorbance).

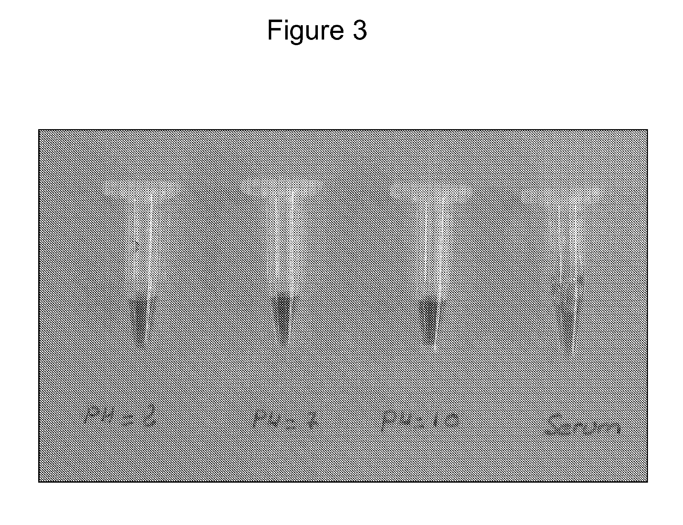

FIGURE 3. Colloidal stability of water soluble Fe₃O₄@Au nanoparticles to different pH and serum.

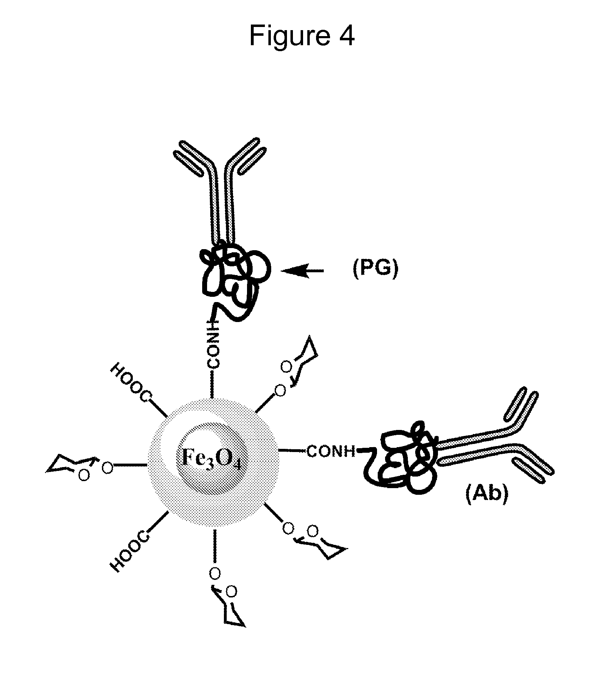

FIGURE 4. Schematic representation of functionalised glyconanoparticles platforms with protein G (PG) and well-oriented conjugated antibody (Ab) Fe₃O₄@Au @PG@Ab.

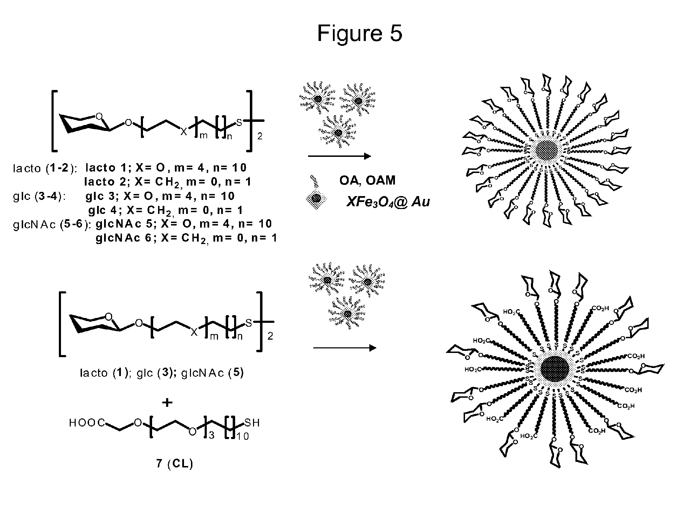

FIGURE 5. Synthesis of water soluble lacto-, gluco- and glucosamine-protected glyconanoparticles and hybrid glyconanoparticules composed of the above carbohydrates and the carboxylic linker 7; OA stands for oleic acid; OAM stands for oleylamine; and CL stands for carboxylic linker.

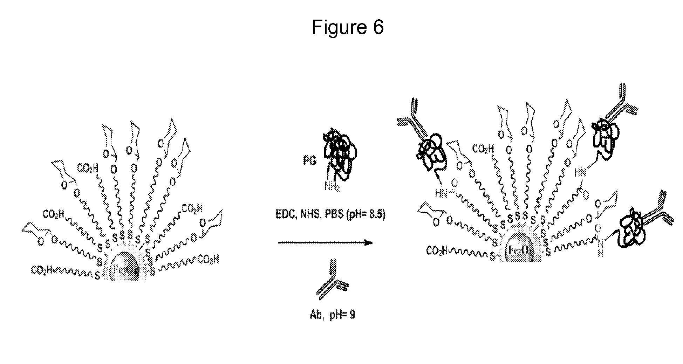

FIGURE 6. Representation of the process for production of magnetic nanoparticles conjugated to protein G (Fe₃O₄@Au @PG) and antibodies (Fe₃O₄@Au @PG@Ab); PG stands for Protein G; EDC stands for 1-Ethyl-3-(3-dimethyllaminopropyl)carbodiimide; NHS stands for N-hydroxisuccinimide; PBS stands for phosphate buffered saline.

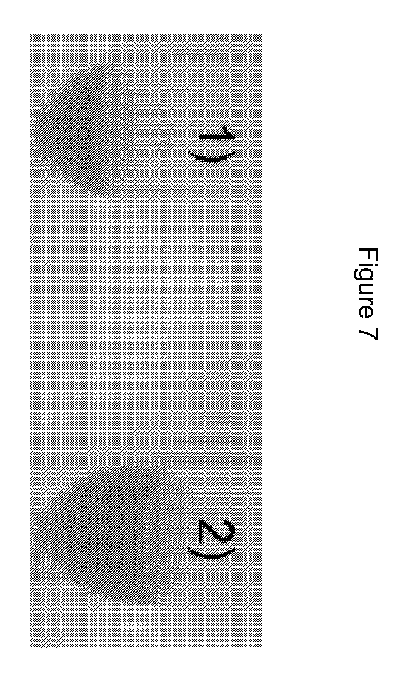

FIGURE 7. Water solubility of 1) lyophilised Fe₃O₄@Au@PG@Ab and 2) lyophilised Fe₃O₄@Au@PG nanoparticles.

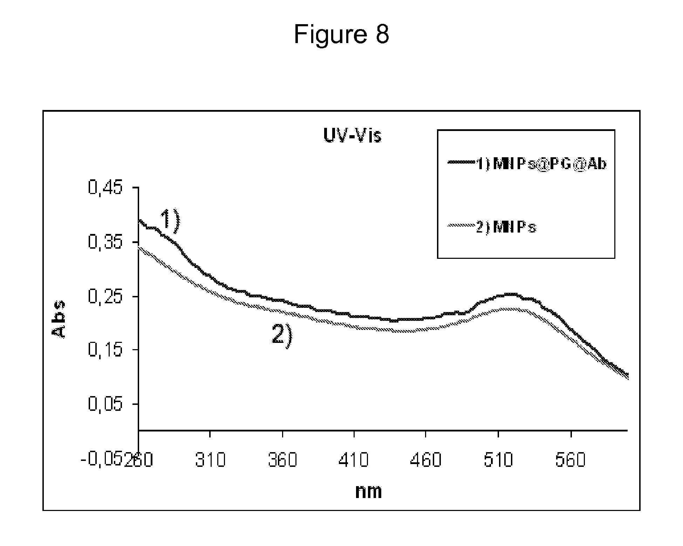

FIGURE 8. UV-Vis spectrum of Fe₃O_{4@}Au@PG@Ab conjugate (curve 1, blue) and Fe₃O₄@Au@PG (curve 2, pink); the surface plasmon resonance due to gold can be clearly seen at about 526 nm (curves 1 and 2); the absorbance of the antibody at 280 nm appears as a slightly shoulder in the antibody bioconjugate solution (curve 1).

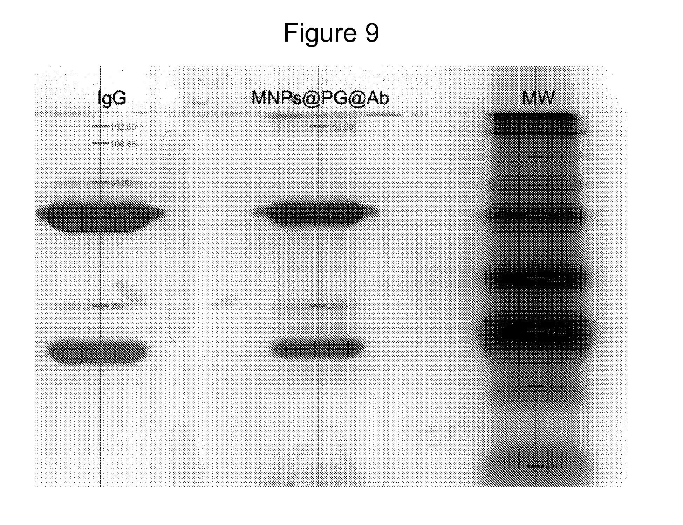

FIGURE 9. SDS-PAGE electrophoresis of the antibody (Ab) (line 1), MNPs@Au@PG@Ab (line 2) and molecular weight markers (line 3); MNPS stands for magnetic nanoparticles; MW stands for Molecular Weight.

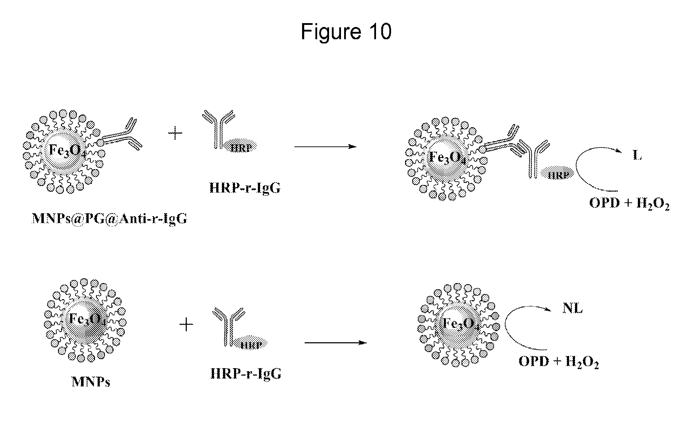

FIGURE 10. Principle of immunoassay for determination of well orientation of the antibody in the conjugate (MNPs@PG@anti-r-IgG); anti-r-IgG stands for goat anti-rabbit IgG; HRP-r-IgG stands for horseradish peroxidase-rabbit-antisheep IgG; OPD stands for 1,2-o-phenylenediamine; L stands for light; NL stands for not light.

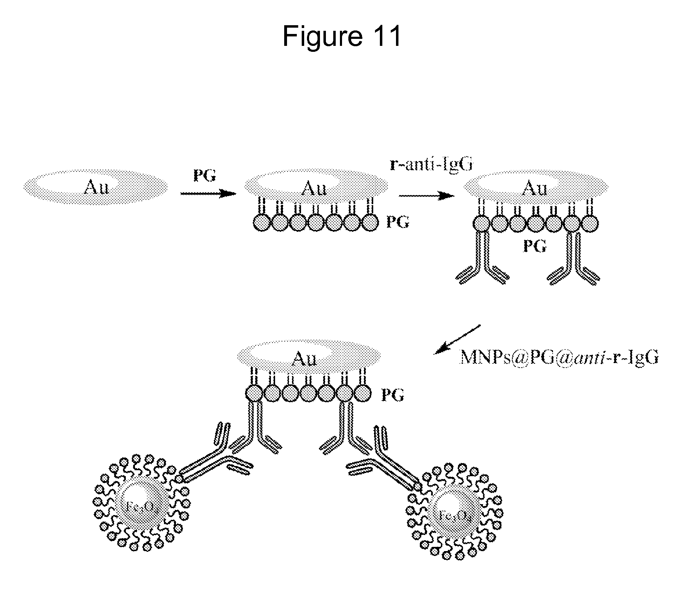

FIGURE 11. Schematic representation of the QCM-D experiments to confirm biofunctionality of the MNPs@PG@anti-r-IgG.

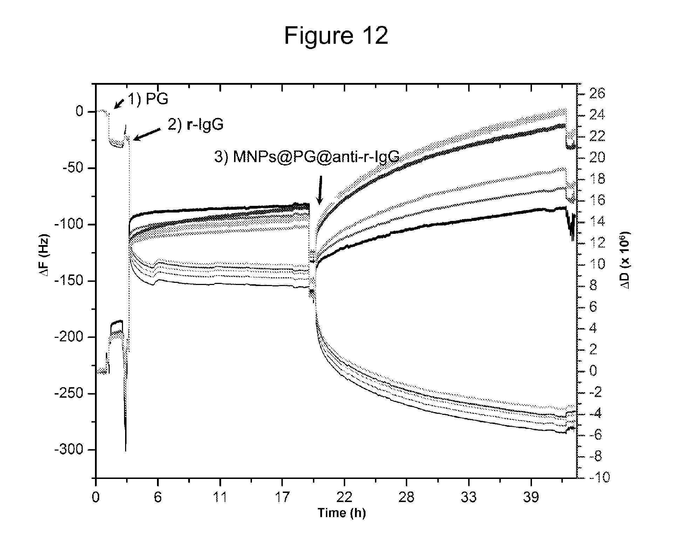

FIGURE 12. QCM data showing functionality of the anti-r-IgG in the conjugate MNPs@PG@anti-r-IgG. The plot shows the change of frequency (Δf, lower graph) and dissipation (ΔD upper graphs) as a function of the time of a gold QCM sensor after the absorption of protein G (step 1), immobilization of the antibody r-IgG (step 2) and specific recognition of MNPs@PG@anti-r-IgG (step 3). Protein G adsorption gives a shift in the frequency lower than the corresponding shift obtained when r-IgG antibody was added. The most strong shift in frequency was observed when the conjugate MNPs@PG@anti-r-IgG was injected. The binding kinetics is slower for these complexes due to slower diffusion. No addition of mass to the sensor was observed when MNPs (without antibody) was injected (data not shown).

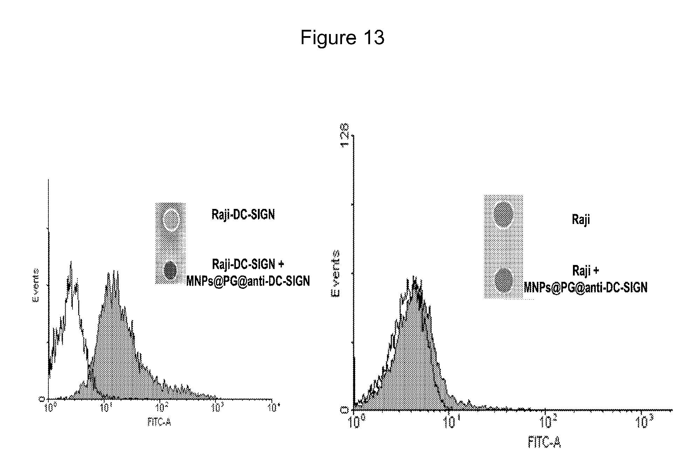

FIGURE 13. Flow cytometer (FC) analysis and T2-weighted images of the specific labelling of Raji DC-SIGN (left) versus Raji (right) by MNPs@PG@anti-DC-SIGN. For both experiments, Raji DC-SIGN and Raji cells were incubated in the presence of MNPs@PG@anti-DC-SIGN for 1 hour at 4°C.
   FC analysis of Raji and Raji+ cells incubated with anti-DC-SIGN-MNPs revealed that rajishowed a higher mean fluorescence emission that the non-transfected Raji-control. The T2-weighted images of the Raji DC-SIGN treated with MNPs@PG@anti-DC-SIGN present a significant contrast enhancement. However, T2-weighted images of Raji cells treated under the same conditions present no differences. As control experiments, untreated cells with media alone were used.

### EXAMPLES

The present invention is more particularly described in the following examples, which are intended to be only illustrative.

### Example 1: Synthesis of 4 nm Fe₃O₄

A thermal decomposition method was followed to synthesize these seeds. Briefly, oleic acid (6 mmol), oleylamine (6 mmol), 1,2-hexadecanediol (10 mmol), and Fe(cetylacetonate)3 (2 mmol) were disolved into 20 ml of phenyl ether. The solution was heated, with vigorous magnetic stirring, to 200°C ( 30 min) and the temperature was risen up to 250 °Cand the mixture was heated for 30 min at this temperature. The resulting solution had a dark brown colour. After cooling to room temperature, 40 ml of ethanol were added into the solution to precipitate the nanoparticles. The resulting Fe₃O₄ nanoparticles were precipitades by adding Ethanol and purified by magnetic sorting out (3 times) and finally resuspended in hexane containing 75 mM of oleic acid and oleylamine.

### Example 2: Preparation of 4nm Fe₃O₄@Au nanoparticles

Oleic acid (1'5 mmol), oleylamine (6 mmol), 1,2-hexadecanediol (12 mmol) and Au(Ac)₃ (0'55 mmol) were added to a hexane solution of Fe₃O₄ magnetic cores (10 ml) and mixed with phenyl ether (30 ml). This solution was heated under vigorous stirring and under Ar atmosphere at 190°C during 90 min. After this procedure the solution changed its colour from dark brown to dark purple. After cooling to room temperature, 40 ml of ethanol were added into the solution to precipitate the nanoparticles. The resulting Fe₃O₄@Au nanoparticles were washed (3 times) by magnetic sorting and finally the nanoparticles were resuspended in hexane containing 75mM oleic acid and 75 mM oleylamine.

### Example 3: Preparation of 13.5 nm MnFe₂O₄@Au nanoparticles

In the case of the MnFe₂O₄ nanoparticles, the gold-coating procedure followed was that of Fe₃O₄ nanoparticles_with the only variation that the gold reagent was added, drop by drop over the preheated mixture reaction. The resulting MnFe₂O₄@Au nanoparticles were separated magnetically, washed 3 times with ethanol, and kept resuspended in 75 mM oleic acid and 75 mM oleylamine hexane until further use.

### Example 4: Preparation of 12.5 nm CoFe₃O₄@Au nanoparticles

In the case of the CoFe₂O₄ nanoparticles, the gold-coating procedure followed was that of MnFe₂O₄._The resulting CoFe₂O₄@Au nanoparticles were separated magnetically, washed 3 times with ethanol, and kept resuspended in 75 mM oleic acid and 75 mM oleylamine hexane until further use.

### Example 5: Monitorization of the gold coatinq process of the nanoparticles

This process of coating was monitored using UV-Vis spectroscopy and Transmission Electron Microscopy (TEM). Before the addition of the gold, the extinction properties of the superparamagnetic particles (Fe₃O₄) were consistent with sub-wavelenght sized dielectric spheres but upon addition of the gold layer onto the metallic core, the extinction spectrum changes hardly appearing a typical plasmon resonance peak (around 520 nm). The coating with Au changes the plasmonic properties of the core/shell structure depending on the gold thickness. In the case of magnetite, coating was performed at three different Fe:Au ratios (1:7, 1:1.75, 1:0.5 eqs.) with consequent decrease in the Au layer thickness. UV-Vis spectra series from Fe₃O₄@Au prepared at different ratios was carried out. The Fe₃O₄@Au nanoparticles at a proportion Fe:Au 1:0.5 have an absorption peak at 513 nm. Deposition of more Au on the as-seed nanoparticles leads to a red-shift of the absorption peak to 526 for a Fe/Au ratio 1:1.75 and to 530 nm for 1:7, respectively. The shift towards the red when the dimensions of the Au coating increased.

### Example 6: Synthesis of water soluble hybrid nanoparticles Fe₃O₄@Au (glycoconjugates 2, 3 or 4)₅₀ (linker 7)₅₀

In a general procedure, 1 mL of a solution of Fe₃O₄@Au nanoparticles (∼0.011 mmol) were added 5 mL of ethanol. The nanoparticles were precipitated and separated by magnetic decantation. The process was repeated two times more. Finally the nanoparticles were suspended in 5 mL of hexane, placed in a falcon tube and a solution of 0.06 mmol of the glycoconjugates (2, 3 or 4) and 0.06 mmol of the mixed linker 7 in 5 mL of water was added. After 5 min, 20 mg of NaBH4 (0.5 mmol) was also added.

The tube was shaken overnight and the transfer reaction was followed by the change of colourness in the hexane phase. With the C5 modified glycoconjugates (2, 4 and 6), it was necessary to allow the slow evaporation of the hexane to force the molecules into water. The coloured aqueous phase was passed through a 0.2-µm Nylon syringe filter to remove possible larger micelles. The nanoparticles were first lyophilized, dissolved in water, precipitated by adding methanol, separated by magnetic decantation, washed twice with methanol and finally purified by dialysis. Aqueous solution (5 mL) was loaded into 5-10 cm segments of SnakeSkin® pleated dialysis tubing (Pierce, 10000 MWCO), placed in a 3 L beaker of water, and stirred slowly, recharging with distilled fresh water every 3-4 hours over the course of 72 h. The resulting solution containing glycoconjugated Fe304@Au was lyophilised to afford the glyconanoparticles as a black powder (12 mg).

The glyconanoparticles were characterized by Low and High-resolution Transmission Electron Microscopy. The ratio of the ligands on the resulting hybrid corresponding magnetic nanoparticles was determined by integration of the 1 H-NMR spectrum of the mixture (glycoconjugate/mixed linker) before and after ligand exchange. The presence of the ligands on the shell was confirmed by treated glyconanoparticles with NaCN and registered 1 H-NMR spectra of the destroyed nanoparticles.

### Example 7: Covalent coupling of protein G to the water soluble glyconanoparticles of example 5.

Usually, 1'0 mg of lyophilized glyconanoparticles were dissolved in 1 mL of 10 mM PBS pH 7.4. To activate the acidic groups, a solution of 1-Ethyl-3-(3-dimethylaminopropyl) carbodiimide/N-hydroxysuccinimide (EDC/NHS) in relation 1/1.5 in PBS was added into the solution of the glyconanoparticles (to a final concentration of 40 µg EDC/mL). This mixture is allowed to shake for 90 min, and then is diluted to 2 ml. In this moment, a solution of 1 mg/ml of Protein G (161 mg; relationship 1/5 glyconanoparticles /Protein G) is added to the mixture, and it is shaken for 3 hours. The purification of this product is done by centrifuging right after the three hours (to avoid adsorption and therefore the formation of nets), three times at 16400 rpm for 90 min at 4 °C. The resulting pellet containing XFe₂O₄@Au@PG nanoparticles is resuspended in 10 mM PBS pH 7.4 and kept frozen until further use.

### Example 8: Coupling of an antibody (IqG) to the protein G

Ten µL (aprox. 7.10-10 mol of protein G) of XFe₂O₄@Au@PG solution were incubated for 6 hour at 10°C with 136 µL of a solution 400 µg/ml of any IgG (aprox.3'5·10-10 mol, ratio glyconanoparticles /IgG ½.5). The sample was centrifuged (three times) to separate the XFe₂O₄@Au@PG@Ab from the unbounded antibody at 16400 rpm for 90 min, resuspended in PBS, and frozen until further use.

### Example 9: Characterisation of the biofunctionilised alyconanoparticles Fe₃O₄@Au@PG coniugated with the antibody

For the characterisation of Fe₃O₄@Au@PG@Ab nanoparticles the UV-Vis spectra was recorded from the Fe₃O₄@Au@PG colloidal solution before (curve 1, pink) and after (curve 2, blue) conjugation with the antibody (overall protein concentration in the solution 10⁻⁷ M). To determine the presence of the antibody in the nanoparticle a SDS-PAGE electrophoresis gel was also carried out. The constructions were purified by centrifugation until washings did not have free antibody (by SDS-page analysis). In this kind of gels, the nanoparticle could not enter because of its big size, but the bands of the antibody were clearly visible (two bands at ∼25 and ∼50 KDa because of the reductive treatment of the samples previous to the seeding). Also the amount of antibody present in the washings, after the incubation, was measured using the Bradford method. The concentration of the IgG in the nanoparticle calculated by mass balance was around 96%. The binding capacity of IgG on the beads was estimated as 4.5 nmol/mg (IgG/nanoparticles) by absorption spectrometry. The next important point was to determine the biofunctionality of the antibodies attached to the magnetic nanoparticles.

### Example 10: Immunoassay of MNPs

Goat anti-rabbit IgG (primary antibody) labelled nanoparticles were washed with 0.01 M PBS (pH 7.4) containing 0.05 % Tween 20. After incubation with HRP- rabbit antisheep IgG (secondary antibody) for 3 hours at room temperature, the nanoparticles were centrifuged at 16.400 r.p.m. for 90 min three times. A 100 µl aliquot of the supernatant that contained the unbound HRP-labelled rabbit antisheep IgG was treated with 50 µl 2.2 mM 1,2-o-phenylenediamine hydrochloride (OPD) containing 0.012 % 30% hydrogen peroxide at room temperature and in the dark for 30 min. The reaction was stopped by adding 50 µl H₂SO₄ and the absorption was measured at 492 nm. Ninety two percent of the secondary antibody used was recognised by the biofunctionalised nanoparticles and the amount of non-specifically bound HRP-labelled rabbit anti-sheep determined was found to be negligible.

### Example 11: QCM tests

Gold QCM sensor chips (planar gold-coated polished 5 MHz AT-cut crystals) were used for these measurements. The gold surfaces were thoroughly cleaned immediately before use. The sensors were first sonicated in Cobax solution for 10 min, then rinsed and sonicated in Nanopure water another 10 min, and last subjected to UV-ozone treatment for 20 min. This protocol was repeated three times, and thereafter the chips were immediately mounted in the QCM cells. The QCM cell and tubing were thoroughly rinsed with Hellmanex 1%, and Nanopure water. The experiments were performed by sudden exchange of the solutions in the experimental chamber as follows: after stabilization of the QCM-D baseline first in MilliQ water and then in 10 mM PBS pH 7'4, a protein G solution, 0'05 mg/ml, was added to the clean sensor surface. Once the adsorption reached the steady state, loosely bounded molecules were rinsed off with PBS. After rinsing the surface, a rabbit antisheep IgG solution, 0'1 mg/ml, was injected into the system. The surface was rinsed again with phosphate buffered saline (PBS), and MNPs@PG@*anti*-r-IgG solution was added to the chip. To end, the surface was rinsed again to remove any unbounded nanoparticle complex. The control experiment was performed exactly in the same way but the nanoparticle complex injected was not complete, but it lacked the antibody. All experiments were performed at 37°C, with the temperature being controlled by the instrument.

### Example 12: In vitro labeling of DC-SIGN transfected cell line (Raji+) by using NP with the targeting specific receptor DC-SIGN (CD209)

The Raji line of lymphoblast-like cells, established from a Burkitt's lymphoma, and Raji-DC-SIGN transfectants were grown in RPMI-1640 medium supplemented with 10% fetal calf serum (FCS). Media also contained 2mM L-glutamine and streptomycin/penicillin (100U/mL penicillin and 100 µg/mL streptomycin). For the flow Cytometry Analysis of Raji and Raji DC-SIGN, 2x 10⁵ cells were putted into a special facs tube in 300 µl of medium. After added (1 x PBS 500 µl), the solution was centrifuged at 4 °C (5-10 min) at 440g three times. The cells were incubated with 10 µl of a solution of the MNPs@PG@*anti*-DC-SIGN-Ab (1 µg/ µl of antibody) in 50 µl (final volume) of blocking buffer for 1 h at 4° C. Two washing and incubation with 50 µl of a solution of secondary antibody alexa-Fluor488 rabbit anti-mouse IgG (5 µg/mL) in blocking buffer for 30 min at 4° C. After three washing, the cells were fixed adding 100 µl (2% formaldehyde/PBS 1X) during 10 min at r.t., washed with with 500 µl of PBS 1X and finally the cells were suspended in 300 µl PBS 1X buffer before the measurement by FC.
In vitro MR imaging of Raji and Raji DC-SIGN. Raji and Raji DC-SIGN (10⁴ cells) were putted in an eppendorf in 300 µl of media. Then, we added the conjugate (120 µg, 0.4 µg/ µl in nanoparticle) 1 h at 4° C. After fixing with 4% formaldehyde/PBS solution 30 min at room temperature, the remaining cells were washed and centrifugated, and the resulting cell pellet were mixed with 1% agarose solution to measure by MRI. For T2-weighted MR imaging of in vitro cells the following parameters were adopted: all data were acquired with 256x256 points and a resolution of 250um in plane, with a slice thickness of 1.5mm. The TE values were varied in 16 equally spaced steps ranging from 20ms to 320 ms and TE 5 secs. The images were fitted into Levenberg-Margardt method to calculate T2 values using Bruker's Paravision 5 software.

### Example 13: Measurement of longitudinal T1 and transversal T2 relaxation times of Fe₃O₄@Au, MnFe₂O₄@Au and CoFe₂O₄@Au nanoparticles of the present invention

In order to test the potential of the ferrites coated with gold of the present invention as contrast agents, longitudinal T1 and transversal T2 relaxation times were measured and the calculated relaxivity values r2 were compared with other contrast agents under clinical use (cf. Table 1).

**Table 1:**

| Name | Magnetic Core size (nm) | Total size (nm) | Coating material | r2 [a] [mM-1 S-1] | B (T) |
|---|---|---|---|---|---|
| AMI-25 (Feridex; Endorem) [22] | 5-6 | 80-150 | Dextran | Ca. 100/ 93[b] | 1.5 |
| SHU 555A (Resovist) [23] | ca. 4.2 | ca. 62 | Carbodextran | 151 | 0.47 |
| AMI-227, [24] (Sinerem) | 4-6 | 20-40 | Dextran | 53 | 0.47 |
| CLIO,MION, [25] | ca. 2.8 | 10-30 | Dextran | ca. 69 | 1.5 |
| FeₛO4 @Au | 4 | 12.6 | lactose | 155 | 1.5 |
| MnFe₂O₄@Au | 6 | 20.1 | lactose | 163 | 1.5 |
| CoFe₂O₄@Au | 6 | 19.1 | lactose | 66 | 1.5 |

In general, these new nanoparticles exhibit relaxivity values in the range of conventional iron oxide contrast agents. In the case of Fe₃O₄ and MnFe₂O₄ higher values were measured. The relaxivity of our nanocomposites decreases in the order MnFe₂O₄>Fe₃O₄>CoFe₂O₄ (see Table 1).

Finally, these glyconanoparticles were subsequently tested for its compatibility with living cells in vitro. No nanoparticles resulted cytotoxic for the tested cell lines (C6 Rat glioma and C8-D1A mouse astrocite). The cells were incubated 3 and 5 days in the presence of different concentrations of magnetic glyconanoparticles into a range of 0'01 µg/ml to 100 µg/ml. A different behaviour was observed in terms of uptake cellular as function of the chemical nature of their shell. For example, at short incubation time (1 hour) and 37°C, the nanoparticles modified with carbohydrates are successfully internalized but nanoparticles decorated only with the carboxylic linker were not up taken by cells.

## Claims

1. Gold-coated nanoparticles comprising:
a) a magnetic core of XFe₂O₄ wherein X is a metal selected from the group consisting of Fe, Mn, and Co;
b) carbohydrates covalently coupled through a spacer to the gold-coated nanoparticle, wherein said spacer has a tiol linking it to the nanoparticle; and,
c) an immunoglobulin-binding protein coupled to the gold-coated nanoparticle through an amphiphilic molecule, comprising a functional group capable of coupling the protein and through a thiol group which is attached to the gold coating of the nanoparticle.

2. The nanoparticles according to claim 1, wherein the magnetic core is Fe₃O₄.

3. The nanoparticles according to claim 2, wherein the magnetic core has a size between 4 and 12 nm and the gold coated magnetic core has a size between 6 and 14 nm.

4. The nanoparticles according to claim 1, wherein the magnetic core is selected from MnFe₂O₄ and CoFe₂O₄.

5. The nanoparticles according to claim 4, wherein the magnetic core has a size between 6 to 12 nm and the gold coated magnetic core has a size between 12 and 18 nm.

6. The nanoparticles according to any of the claims 1-5, wherein the amphiphilic molecule has the following formula: X-(EG)-(CH₂)₁₁-SH, wherein X is the functional group and EG is ethylene glycol.

7. The nanoparticles according to any of the claims 1-6, wherein the functional group of the amphiphilic molecule is selected from the group consisting of a carboxylic acid, an isothiocyanate, and a maleimide.

8. The nanoparticles according to any of the claims 1-7, wherein the carbohydrate is selected from the group consisting of lactose, glucose, and glucosamine.

9. The nanoparticles according to claim 8, wherein the carbohydrate and the spacer have one of the following formula:

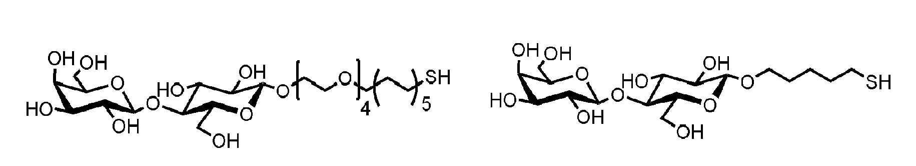

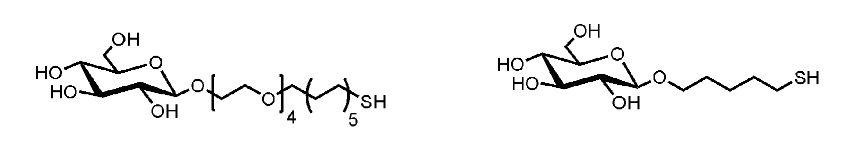

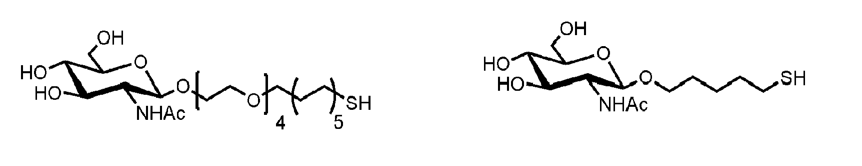

10. The nanoparticles according to any of the claims 1-9, wherein the immunoglobulin-binding protein is protein G or protein A.

11. The nanoparticles according to claim 10, wherein the protein G, the protein A, or the linker molecule have a fluorophore.

12. The nanoparticles according to any of the claims 1-11, wherein an antibody is coupled to the immunoglobulin-binding protein.

13. A method of preparing the gold-coated nanoparticles as defined in any of the claims 1-11, comprising:
(a) coating a magnetic core of XFe₂O₄ wherein X is a metal selected from the group consisting of Fe, Mn, and Co by contacting the core with Au(Acetyl)₃ in the presence of at least one surfactant;
(b) mixing a non-polar solution of the gold-coated nanoparticles obtained in step (a) with an aqueous solution of glycoconjugates and amphiphilic molecules, the amphiphilic molecules comprising a functional group capable of coupling the protein and a thiol group which is attached to the gold coating of the nanoparticle; and,
(c) covalently coupling an immunoglobulin-binding protein to the nanoparticles.

14. The method of claim 13, further comprising coupling an antibody to the immunoglobulin-binding protein to yield the nanoparticles of claim 12.

15. Use of the nanoparticles as defined in any of the claims 1-12, as contrast agents by MRI.

16. Use of the nanoparticles as defined in claim 11, as detection agents of non-labelled antibodies.

17. A kit comprising any of the nanoparticles as defined in any of the claims 1-12.
